# EUROPEAN PATENT SPECIFICATION

(11) **EP 3 841 512 B1**
(45) Date of publication and mention of the grant of the patent: **20.05.2026**
(21) Application number: 18930933.9
(22) Date of filing: 23.08.2018
(51) Int. Cl.: G06T 19/20

(54) **HYBRID METHOD OF ACQUIRING 3D DATA USING INTRAORAL SCANNER**
HYBRIDES VERFAHREN ZUR ERFASSUNG VON 3D-DATEN MITTELS INTRAORALEM SCANNER
PROCÉDÉ HYBRIDE PERMETTANT D'ACQUÉRIR DES DONNÉES 3D À L'AIDE D'UN SCANNER INTRA-BUCCAL

(43) Date of publication of application: 30.06.2021
(73) Proprietor: Carestream Dental Technology Shanghai Co., Ltd., Shanghai 201206 (CN)
(72) Inventor: ZHOU, Yu, Shanghai 201206 (CN); CHEN, Qinran, Shanghai 201206 (CN); ZHUANG, Siqi, Shanghai 201206 (CN); LIU, Zhaohua, Shanghai 201206 (CN)
(74) Representative: Hoffmann Eitle
(86) International application number: PCT/CN2018/101891
(87) International publication number: WO 2020/037588

(56) References cited:
- EP-A1- 2 742 857
- EP-A2- 2 706 509
- WO-A2-2017/125926
- CN-A- 103 258 070
- CN-A- 106 820 563
- US-A1- 2014 169 648
- US-A1- 2015 320 320
- US-A1- 2017 103 569
- YANG WEI-MIN ET AL: "Automatic Superimposition of Palatal Fiducial Markers for Accurate Integration of Digital Dental Model and Cone Beam Computed Tomography", JOURNAL OF ORAL AND MAXILLOFACIAL SURGERY., vol. 73, no. 8, 1 August 2015 (2015-08-01), US, pages 1 - 10, XP093282755, ISSN: 0278-2391, DOI: 10.1016/j.joms.2015.04.004

## Description

### FIELD OF THE INVENTION

The invention relates generally to intraoral imaging and more particularly to generating and conditioning a 3D model of patient dentition using combined results from intraoral scanning directly from the patient and from an impression.

### BACKGROUND OF THE INVENTION

Intraoral imaging has proven to have significant value as a tool with a diverse range of applications, including use as a diagnostic aid, use for treatment and restoration planning, and use in color shade matching, among other applications. Optical intraoral scans produce contours of dentition objects and are beneficial in improving visualization of teeth, gums, and other intra-oral structures. Surface contour information can be particularly useful for assessment of tooth condition and has recognized value for various types of dental procedures, such as for restorative dentistry.

For orthodontic and other restorative procedures, a model of patient dentition is generated, initially and at various stages of the process. A number of standardized metrics can then be applied to the models for comparison and to track overall progress of the treatment regimen.

In conventional practice, the generated model is formed from an impression obtained from the patient. This standard method is time consuming and can be uncomfortable for the patient, but has been perfected over a number of years so that the materials and methods used, in spite of some drawbacks, provide very suitable results for forming an accurate model of patient dentition.

Thanks to continuing improvement in image quality and response time, 3D intraoral scanners provide tools for acquisition of a digital model that can be readily obtained without the encumbrance of impression techniques and materials. Using the 3D intraoral scanner, a technician or practitioner can obtain accurate volume image information by carefully moving the scanner from tooth to tooth within the mouth of the patient, viewing a display and observing progress of the growing digital data that represents intraoral structures as a mesh or point cloud.

While 3D scanners are convenient and easy to use, however, there is some room for improvement. One particular shortcoming relates to the quality of imaging of localized areas such as the tooth margin. Accurate characterization of the tooth margin is significant for successful preparation and fitting, such as for a crown or implant. 3D intraoral scanners can fail to model the tooth margin to the practitioner's satisfaction, due to illumination constraints, limitations of the imaging optics, and other difficulties. Subgingival tissue can tend to obscure the margin, which is often effectively hidden beneath the soft tissue.

Thus, it can be seen that there would be benefit to a digital imaging approach that more accurately characterizes the tooth margin when forming a 3D model of patient dentition In this context, patent application US2015/320320 A1 discloses the identification and indication in an intraoral scan, of areas of interest that should be rescanned.

Further, reference is made to EP 2 706 509 A2 disclosing an imaging apparatus and a method for displaying a patient's teeth and particularly the maxillary and mandibular arches. The method generates a three-dimensional model of the patient's teeth and displays the generated three-dimensional model. A displayed image shows a cross-sectional view of the teeth according to the position of a cutting plane extending through the three-dimensional model. The relative position of the cutting plane is adjustable according to an operator instruction. The orientation of the displayed cross-sectional view is determined by the position of the cutting plane.

Patent application CN 103 258 070 A discloses the virtual design of a post and core restoration adapted for attachment in a damaged tooth of a patient, involving the virtual match of a 3D scan of the damaged tooth with a digital 3D shape adapted to fit a bore in the damaged tooth.

The publication of Yang Wei-Min et al.: "Automatic Superimposition of Palatal Fiducial Markers for Accurate Integration of Digital Dental Model and Cone Beam Computed Tomography", Journal of Oral and Maxillofacial Surgery, Elsevier, vol. 73, no. 8, August 2015, DOI: 10.1016/ j.joms.2015.04.004, discloses the fusion of 3D scans of plaster dental models with 3D dental models acquired from cone beam computed tomography.

### SUMMARY OF THE INVENTION

In accordance with the present invention, are provided a method for imaging a region of interest of patient dentition as set forth in Claim 1, a method for imaging a region of interest of a dental arch as set forth in Claim 9, an imaging apparatus for imaging a dental arch of a patient as set forth in Claim 10. Further embodiments of the invention are inter alia disclosed in the dependent claims.

It is an object of the present disclosure to advance the art of diagnostic imaging and to address the need for improved 3-D intraoral imaging and scanning, particularly for portions of the teeth related to tooth margin.

These objects are given only by way of illustrative example, and such objects may be exemplary of one or more embodiments of the invention. Other desirable objectives and advantages inherently achieved by the disclosed methods may occur or become apparent to those skilled in the art. The invention is defined by the appended claims.

According to an aspect of the present invention, there is provided a method for imaging a region of interest of patient dentition, the method executed at least in part by a computer system and comprising:
a) acquiring an intraoral scan directly from the patient dentition and forming a 3D digital model of a first surface contour of the patient dentition that includes the region of interest;
b) acquiring a physical impression that includes at least the region of interest;
c) scanning the physical impression and generating a second surface contour of the identified region of interest;
d) modifying the digital model by combining a portion of the second surface contour with the first surface contour over the region of interest;
   and
e) displaying the modified digital model.

### BRIEF DESCRIPTION OF THE DRAWINGS

The foregoing and other objects, features, and advantages of the invention will be apparent from the following more particular description of the embodiments of the invention, as illustrated in the accompanying drawings.

The elements of the drawings are not necessarily to scale relative to each other. Some exaggeration may be necessary in order to emphasize basic structural relationships or principles of operation. Some conventional components that would be needed for implementation of the described embodiments, such as support components used for providing power, for packaging, and for mounting and protecting system optics, for example, are not shown in the drawings in order to simplify description.
FIG. 1 is a schematic diagram that shows a surface contour imaging apparatus for obtaining a 3D view from a succession of reflectance images according to an embodiment of the present disclosure.
FIG. 2 is a schematic diagram showing an imaging apparatus that can operate as a video camera for polychromatic reflectance image data capture as well as a scanner for executing related projecting and imaging functions.
FIGs. 3 and 4 show point cloud images generated from a succession of structured light images.
FIG. 5 is a logic flow diagram that shows a sequence for forming a digital model of a dental arch according to an embodiment of the present disclosure.
FIG. 6 shows an exemplary 3D mesh for scanned content.
FIG. 7A shows the tooth mesh with the ROI highlighted.
FIG. 7B shows an on-screen interface for ROI identification.
FIG. 8 shows a partial impression obtained from the patient.
FIG. 9 shows a portion of a scanned impression.
FIG. 10 shows merged surface contour output with a margin area highlighted.
FIG. 11 shows merged surface contour output with highlighting removed.
FIG. 12 is a logic flow diagram showing a sequence for bite registration using a combination of intraoral scanning and scanned impressions according to an example not forming part of the invention.
FIG. 13 shows the mesh corresponding to each arch.
FIG. 14A shows positioning of an impression for bite registration.
FIG. 14B shows an example of an impression acquired for bite registration.
FIG. 15 shows top and bottom surfaces of an example impression of a patient's occlusal surfaces.
FIG. 16 shows registration of the bite impression data from occlusal surfaces with scanned data from the upper and lower arch.
FIG. 17 shows the output of bite registration for an example not forming part of the invention, using the bite impression data.

### DETAILED DESCRIPTION OF THE INVENTION

The following is a detailed description of the preferred embodiments, reference being made to the drawings in which the same reference numerals identify the same elements of structure in each of the several figures.

Where they are used in the context of the present disclosure, the terms "first", "second", and so on, do not necessarily denote any ordinal, sequential, or priority relation, but are simply used to more clearly distinguish one step, element, or set of elements from another, unless specified otherwise.

As used herein, the term "energizable" relates to a device or set of components that perform an indicated function upon receiving power and, optionally, upon receiving an enabling signal.

In the context of the present disclosure, the term "optics" is used generally to refer to lenses and other refractive, diffractive, and reflective components or apertures used for shaping and orienting a light beam. An individual component of this type is termed an optic.

In the context of the present disclosure, the term "scattered light" is used generally to include light that is reflected and backscattered from an object.

In the context of the present disclosure, the terms "viewer", "operator", and "user" are considered to be equivalent and refer to the viewing practitioner, technician, or other person who may operate a camera or scanner and may also view and manipulate an image, such as a dental image, on a display monitor. An "operator instruction" or "viewer instruction" is obtained from explicit commands entered by the viewer, such as by clicking a button on the camera or scanner or by using a computer mouse or by touch screen or keyboard entry.

In the context of the present disclosure, the phrase "in signal communication" indicates that two or more devices and/or components are capable of communicating with each other via signals that travel over some type of signal path. Signal communication may be wired or wireless. The signals may be communication, power, data, or energy signals. The signal paths may include physical, electrical, magnetic, electromagnetic, optical, wired, and/or wireless connections between the first device and/or component and second device and/or component. The signal paths may also include additional devices and/or components between the first device and/or component and second device and/or component.

In the context of the present disclosure, the term "camera" relates to a device that is enabled to acquire a reflectance, 2-D digital image from reflected visible or NIR light, such as structured light that is reflected from the surface of teeth and supporting structures.

The general term "scanner" relates to an optical system that projects a scanned light beam to the tooth surface and provides image content based on the resulting scattered or reflected light content. According to some intraoral imaging embodiments, the terms "camera" and "scanner" may be considered equivalent.

The term "set", as used herein, refers to a non-empty set, as the concept of a collection of elements or members of a set is widely understood in elementary mathematics. The terms "subset" or "partial subset", unless otherwise explicitly stated, are used herein to refer to a non-empty proper subset, that is, to a subset of the larger set, having one or more members. For a set S, a subset may comprise the complete set S. A "proper subset" of set S, however, is strictly contained in set S and excludes at least one member of set S. A "partition of a set" is a grouping of the set's elements into non-empty subsets so that every element is included in one and only one of the subsets. Two sets are "disjoint" when they have no element in common.

The term "subject" refers to the tooth or other portion of a patient that is being imaged and, in optical terms, can be considered equivalent to the "object" of the corresponding imaging system.

In the context of the present disclosure, the terms "viewer", "operator", and "user" are considered to be equivalent and refer to the viewing practitioner or other person who may acquire, view and manipulate a dental or medical image on a display monitor.

In the context of the present disclosure, the terms "point cloud", "mesh", "3D surface image", and "surface image" are used equivalently to describe the digital model of the surface contour that can be formed using a scanner that obtains a series of reflectance images from intraoral or other surfaces. The surface image that provides the digital model is a composite image formed by stitching together individual scans successively acquired for adjacent surface features. Point cloud and mesh are convenient and similar storage and display structures representative of the surface image.

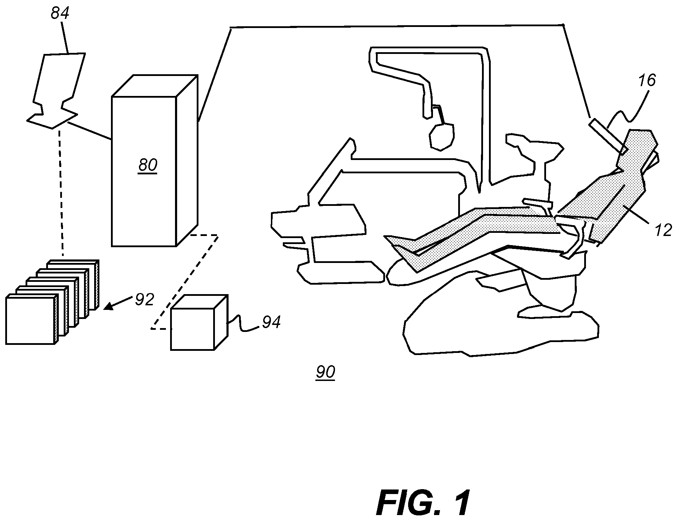

FIGs. 1-4 provide an overview of the intraoral scanner and its use in generating a point cloud or mesh 150 that shows the contour of the scanned surface.

The schematic diagram of FIG. 1 shows a surface contour imaging apparatus 90 that can be used for obtaining 3D content for model generation from a succession of reflectance images according to an embodiment of the present disclosure. A camera 16, typically a hand-held digital camera, a color depth camera, handheld 3D scanner, or intra-oral 3D scanner, is scanned through the mouth of patient 12 for acquiring a set having multiple reflectance images and associated depth information. A control logic processor 80, in signal communication with camera 16, obtains image data from camera 16 and processes this image data along with depth information in order to generate individual 3D views 92. Control logic processor 80 then combines the scanned 3D views in order to generate, store, and optionally render on a display 84, a composite 3D model surface 94. Error detection and correction logic on control logic processor 80 can then operate to identify mis-matched or poorly matched 3D views 92 and to generate and display a corrected composite 3D surface 94. Display 84 is also in signal communication with logic processor 80.

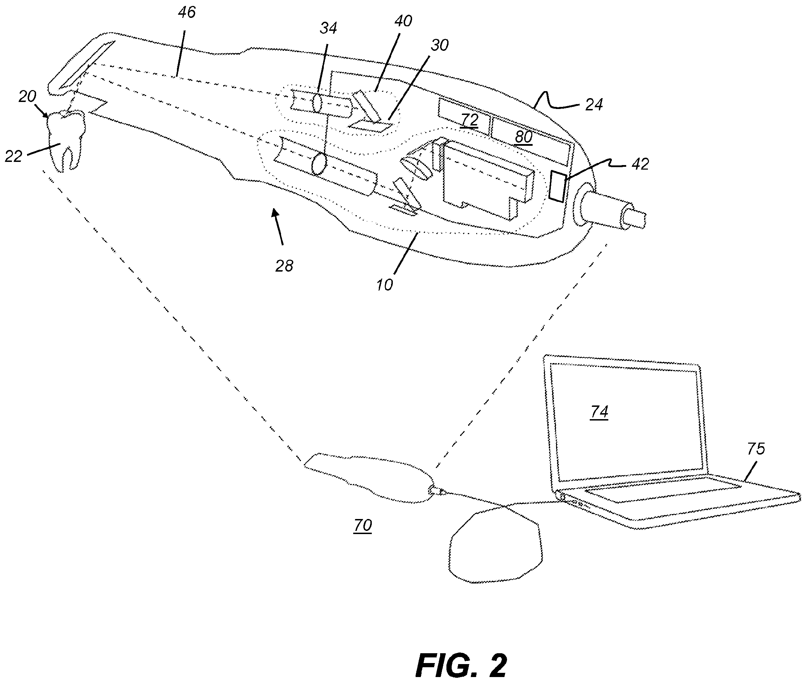

FIG. 2 is a schematic diagram showing an imaging apparatus 70 that can operate as a video camera 24 for polychromatic reflectance image data capture as well as a scanner 28 for executing related projecting and imaging functions used to characterize surface contour with structured light patterns 46. A handheld imaging apparatus 70 uses a video camera 24 for image acquisition for both contour scanning and image capture functions according to an embodiment of the present disclosure. Control logic processor 80, or other type of computer that may be part of camera 24, controls the operation of an illumination array 10 that generates the structured light and directs the light toward a surface position and controls operation of an imaging sensor array 30. Image data from surface 20, such as from a tooth 22, is obtained from imaging sensor array 30 and stored as video image data in a memory 72. Imaging sensor array 30 is part of a sensing apparatus 40 that includes an objective lens 34 and associated elements for acquiring video image content. Control logic processor 80, in signal communication with camera 24 components that acquire the image, processes the received image data and stores the mapping in memory 72. The resulting image from memory 72 is then optionally rendered and displayed on a display 74, which may be part of another computer 75 used for some portion of the processing described herein. Memory 72 may also include a display buffer. One or more sensors 42, such as a motion sensor, can also be provided as part of scanner 28 circuitry.

In structured light imaging, a pattern of lines or other shapes is projected from illumination array 10 toward the surface of an object from a given angle. The projected pattern from the illuminated surface position is then viewed from another angle as a contour image, taking advantage of triangulation in order to analyze surface information based on the appearance of contour lines. Phase shifting, in which the projected pattern is incrementally shifted spatially for obtaining additional measurements at the new locations, is typically applied as part of structured light imaging, used in order to complete the contour mapping of the surface and to increase overall resolution in the contour image. By way of example and not limitation, use of structured light patterns for surface contour characterization is described in US 2013 / 0120 532 A1 and US 2013 / 0 120 533 A1, both entitled "3D INTRAORAL MEASUREMENTS USING OPTICAL MULTILINE METHOD".

### Digital model generation

By knowing the instantaneous position of the camera and the instantaneous position of the line of light or other illumination field within an object-relative coordinate system when the image was acquired, a computer and software can use triangulation methods to compute the coordinates of numerous illuminated surface points relative to a plane. As the plane is moved to intersect eventually with some or all of the surface of the object, the coordinates of an increasing number of points are accumulated. As a result of this image acquisition, a point cloud can be generated and used as the surface image to represent the extent of a surface within a volume. The points in the point cloud then represent actual, measured points on the three-dimensional surface of an object. A mesh can alternately be constructed from the same surface acquisition, represented as vertices for congruent polygonal faces (typically triangular faces) that characterize the surface shape. The full 3D digital model can then be formed by combining the surface contour information provided by the surface image in point cloud or mesh form with polychromatic image content obtained from a camera, such as camera 24 of FIG. 2.

Polychromatic image content can be provided in a number of ways, including the use of a single monochrome imaging sensor with a succession of images obtained using illumination of different primary colors, one color at a time, for example. Alternately, a color imaging sensor could be used.

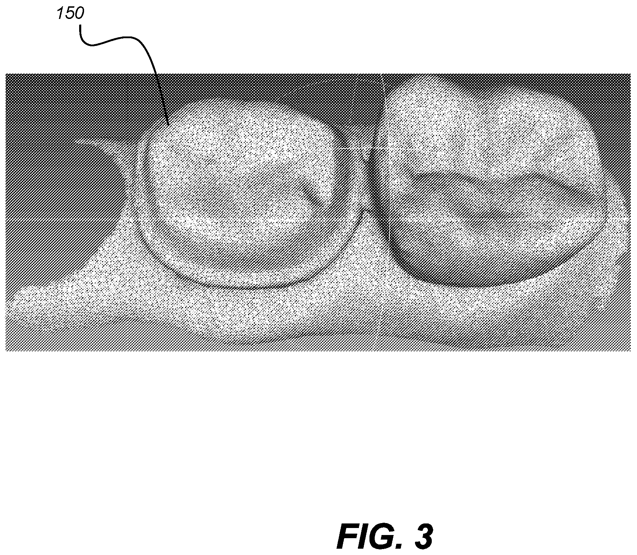

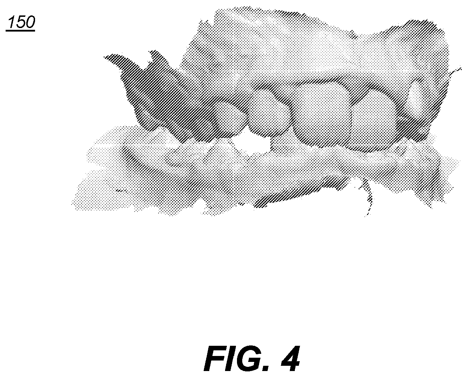

Image processing at control logic processor 80 can generate a digital contour surface model using line scan data from structured light imaging, or using point cloud or mesh data or other volume image data, including video image data. By way of example, FIGs. 3 and 4 show a point cloud or mesh as a surface image 150 generated from a succession of structured light images or other sequence of reflectance images. Further processing of the point cloud content can be used to generate an alternative contour surface model as a mesh, with points of the generated surface serving as vertices of the mesh, as described previously.

It should be noted that other types of reflectance imaging can be used for obtaining intraoral surface contour data used to form the digital model. Surface contour information can be obtained using time-of-flight imaging or range imaging methods, such as structure-from-motion processing, for example.

The conventional method for 3D modeling of teeth and gums of a patient's dentition is a dental impression, which provides a negative imprint of the intraoral features for the complete dental arch. The process of obtaining the impression body requires depositing a soft impression, putty-like material into the patient's mouth and instructing the patient to bite down and hold the bite position for a few minutes while the material hardens to an elastic solid state. Careful removal of the hardened impression material then obtains the physical impression or impression body as a negative imprint that can be used as a mold for forming a 3D model for the corresponding dental arch. While the process is not itself painful or invasive, it can be an unwelcome ordeal and uncomfortable to the patient. Impression techniques can be particularly accurate for reproducing complex curvatures and abrupt surface transitions such as at the tooth cervical margin.

The use of the 3D intraoral scanner or camera allows the practitioner to acquire 3D contour data for the patient without the need for impression materials and apparatus and without the complications and discomfort of conventional impression practice. However, as was noted previously in the background material, it can be difficult to obtain intraoral scan data that reliably and accurately characterizes the tooth margin. There may also be other specific areas of patient dentition, which may be highly patient-specific, for which direct intraoral imaging may not obtain sufficient data to meet the accuracy expectations of the practitioner for generating a 3D digital surface model. Imaging problems can result from insufficient light or excessive light. For example, it can be difficult to direct sufficient light to interproximal features or to obtain enough reflected light from these features for accurate identification of the surface contour. Too much reflected light, such as from a highly reflective ceramic material within the mouth, can also prevent accurate surface characterization. Embodiments of the present disclosure address shortcomings of conventional intraoral scanning that relate to 3D imaging of the tooth margin, and other specific areas, by combining the 3D contour data for the arch, acquired as the surface image from the intraoral scanner, with localized information obtained from scanning a partial impression, that is, an impression that is formed from only a portion of the dental arch.

As noted previously in the background material and shown in the exemplary images of FIGs. 3 and 4, the intraoral scanner provides a capable tool for imaging the surface of intraoral features. There can be cases, however, where the scanner images fall short of providing the surface contour information and precise imaging of the tooth margin or other localized feature is needed. Embodiments of the present disclosure use a hybrid approach to intraoral imaging that, for an identified region of interest (ROI), complements the conventional 3-D intraoral scan with additional scan data acquired from an intraoral impression.

### Hybrid imaging for improved ROI content

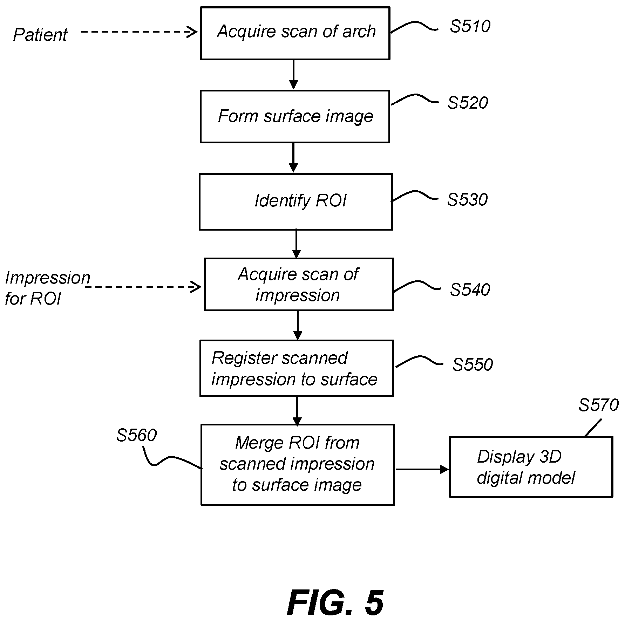

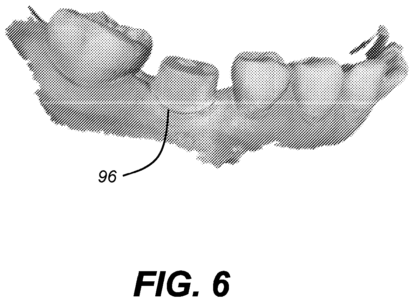

The logic flow diagram of FIG. 5 shows a sequence for dental arch imaging to form a digital model according to an embodiment of the present disclosure. In an intraoral scanning step S510, the intraoral scan of the dental arch is executed. A surface image generation step S520 then generates an initial digital model as a 3D mesh, point cloud, or other 3D surface representation for the scanned content, as shown in the example of FIG. 6. A margin 96 for a preparation is shown.

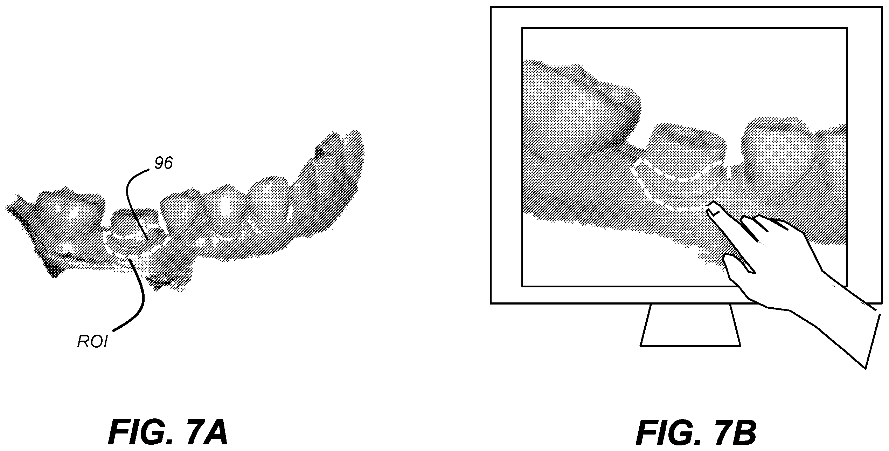

With the surface image generated, an optional ROI identification step S530 then identifies a region of interest (ROI) within the scanned content, as shown highlighted by outline in FIG. 7A. The ROI can be identified by the practitioner or technician using one or more operator instructions entered on-screen on the operator interface. Alternately, the ROI can be automatically detected, such as using pattern recognition software utilities known to those skilled in the image processing arts. In the example of FIGs. 7A and 7B, the ROI is a preparation area with a margin 96. The ROI can be traced or outlined or otherwise highlighted using display screen utilities, such as by designating the area with a brush tool, for example. FIG. 7B shows use of a touch screen interface as one method for entry of instructions for manual ROI identification by the operator.

Automated methods for ROI identification can use various segmentation or pattern-recognition utilities, for example.

Identification of the ROI by the operator or using automated pattern recognition is optional. The ROI can be identified in the surface image from the intraoral scan of the dental arch or, alternately, from the scanned impression acquired in subsequent step S540. According to an alternate embodiment of the present disclosure, the ROI can be defined by default, using the boundary of the scanned impression.

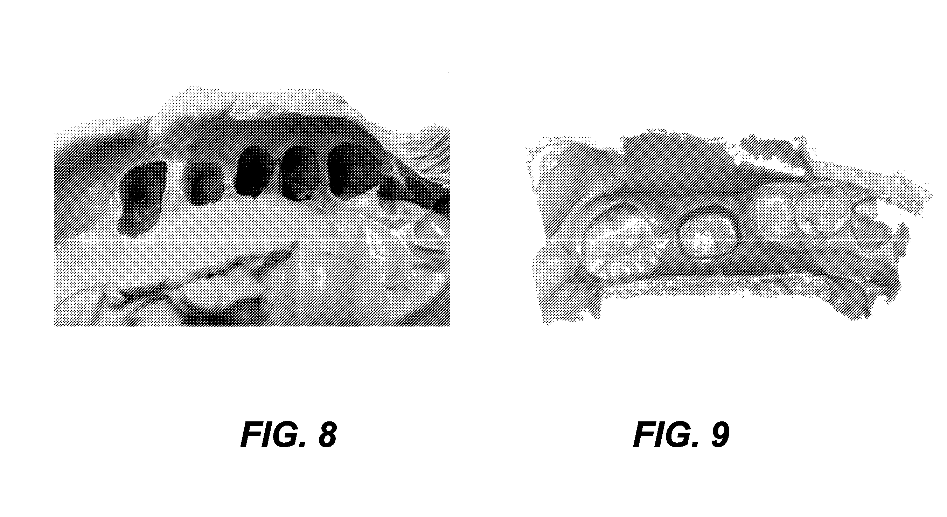

An impression that includes the margin or other ROI is obtained and scanned in an impression scanning step S540. A full impression of the complete dental arch is not needed; only a partial impression is used for this step. The partial impression includes the ROI and can further include sufficient neighboring features to facilitate subsequent registration processing. By way of example, FIG. 8 shows a partial impression obtained from the patient. The impression forms a negative model that can be scanned using the intraoral scanner or other suitable scanning apparatus, such as a desktop scanner, for example. The negative imprint obtained from the impression body can then be digitally inverted to form a positive model. FIG. 9 shows a portion of a scanned imprint from an impression.

A registration step S550 then registers the scanned impression, or at least the portion of the scanned impression that includes the ROI, to the mesh or other type of surface image generated from the intraoral scan. Registration can use a feature matching algorithm or, alternatively, an iterative closest point (ICP) algorithm, or a combination of these two approaches, for example.

A merge step S560 then merges the contour data from the scanned impression of the ROI with the surface image formed from the intraoral scan. Where the ROI is clearly identified, merge step S560 can simply discard the identified nodes of the intraoral scan within the ROI region and substitute the corresponding points or nodes from the registered scanned impression. Where the ROI is only positionally identified, other substitution from the impression data can be performed. For example, nodes of the first model within a given distance of the ROI, such as within 100 microns, can be replaced by the impression data. Other methods for merger or combination of nodes can alternately be applied, including averaging, for example.

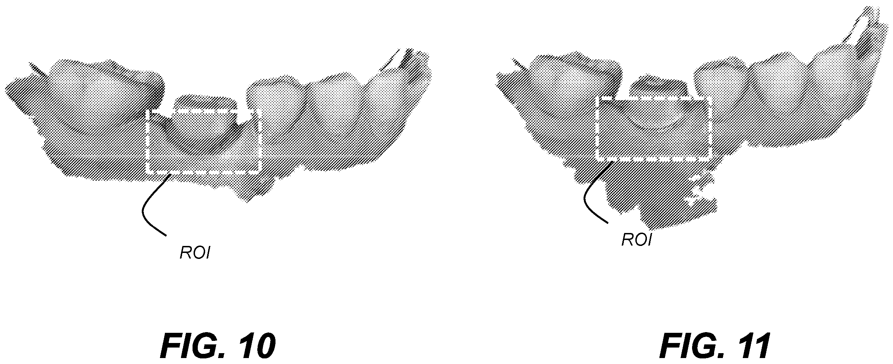

FIG. 10 shows merged surface contour output with a margin area highlighted. FIG. 11 shows merged surface contour output with highlighting removed.

The resultant hybrid digital model can then be rendered to a display in a display step S570. The displayed 3D digital model can optionally indicate the region of interest, such as outlined or otherwise highlighted.

### Hybrid imaging for improved bite registration (not forming part of the invention)

Previous description focused on using a hybrid scanning approach for localized ROI content. The Applicants have also found that complementing the intraoral scan with data from a scanned physical impression can also have value for improved imaging related to bite registration.

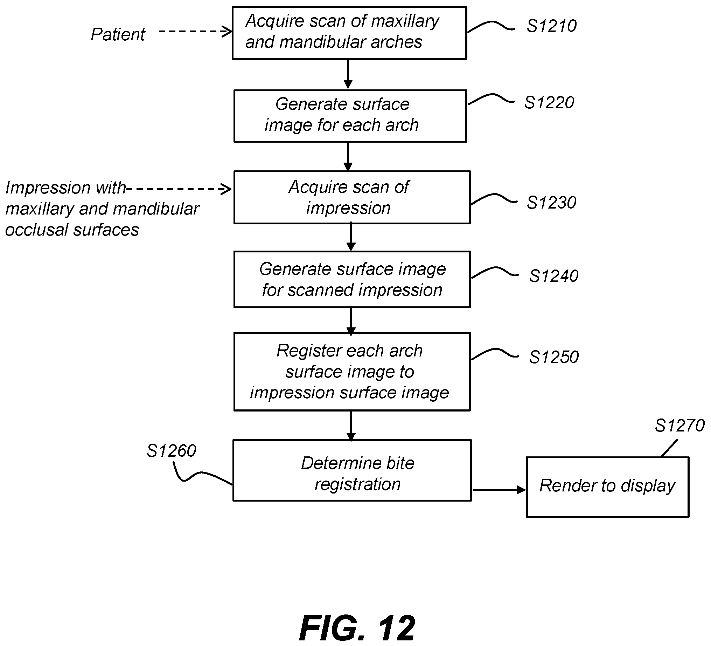

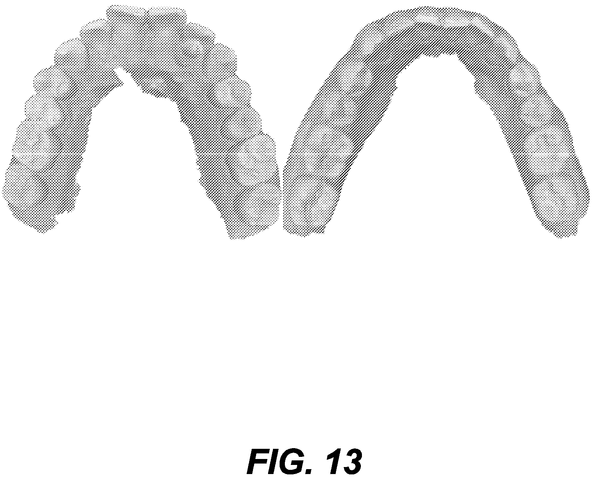

FIG. 12 is a logic flow diagram showing a sequence for bite registration using a combination of intraoral scanning and scanned impressions according to an example not forming part of the invention. In an intraoral scanning step S1210, the practitioner or technician acquires scans for characterizing the surface contour of each arch. A surface image generation step S1220 forms a 3D model, generating the mesh, point cloud, or other type of surface image corresponding to each arch, as shown in the example of FIG. 13.

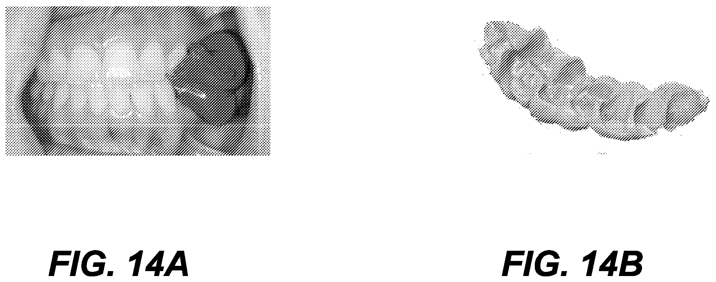

A physical impression of the bite surfaces, acquired at positions that cover only a portion of the intraoral cavity, is then obtained from the patient. For this step, it should be emphasized that only the occlusal surface is needed for the bite registration process of FIG. 12; other portions of the mouth can optionally be included in the impression but are not necessary for this process. Moreover, the acquired physical impression of the occlusion is needed only over some portions of the teeth and not over the full occlusal surface. By way of example, FIG. 14A shows one occlusal surface impression obtained from the patient, with the impression material distributed only over a portion of the patient dentition on one side of the mouth. FIG. 14B shows an exemplary occlusion impression obtained from a portion of the patient's mouth. A second occlusal surface impression could be acquired from the other side of the mouth where useful. Thus, for example, a partial impression from the left side and a partial impression from the right side can be acquired. However, a single impression from one side of the mouth may be sufficient for bite registration in some cases.

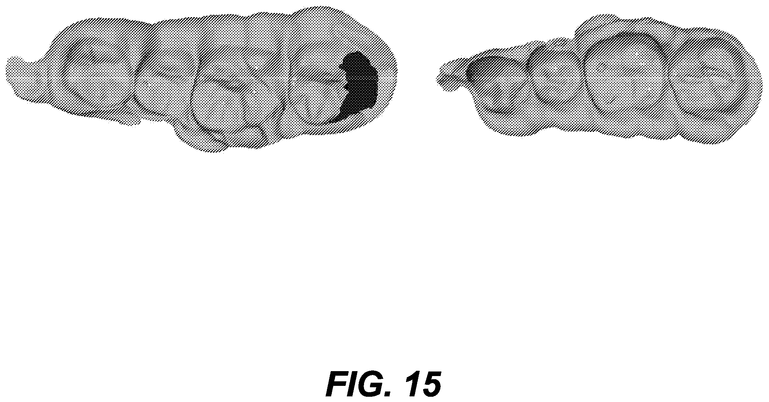

With the occlusal surface impression or impressions obtained, an impression scanning step S1230 is then executed, in which the practitioner or technician scans each obtained occlusal impression using the intraoral scanner or other suitable scanning device, such as a desktop scanner, for example. The occlusion impression is formed from imprints of two facing or opposing bite surfaces, one maxillary, the other mandibular. A subsequent surface image generation step S 1240 then generates a mesh, point cloud, or other type of surface image corresponding to the surface of the acquired occlusion impression. By way of example, FIG. 15 shows maxillary and mandibular surfaces of an example impression of the patient's facing occlusal surfaces.

Still following the FIG. 12 process, a registration step S1250 and a bite determination step S1260 then use the scanned results from the patient and from the impressions in order to accurately characterize the bite registration. In registration step S1250, the surface image for each arch, obtained from previous step S1220 as executed for each arch, is registered with the surface image for the scanned impression that was generated in step S1240. This registration arranges the upper and lower arches in the spatial relationship that is indicative of relative arch position and corresponding bite registration, as identified in bite registration determination step S1260. Registration geometry can be computed using the generated surface image or obtained using a number of methods well known to those skilled in the image processing art. A display step S1270 renders the bite registration as output to a display.

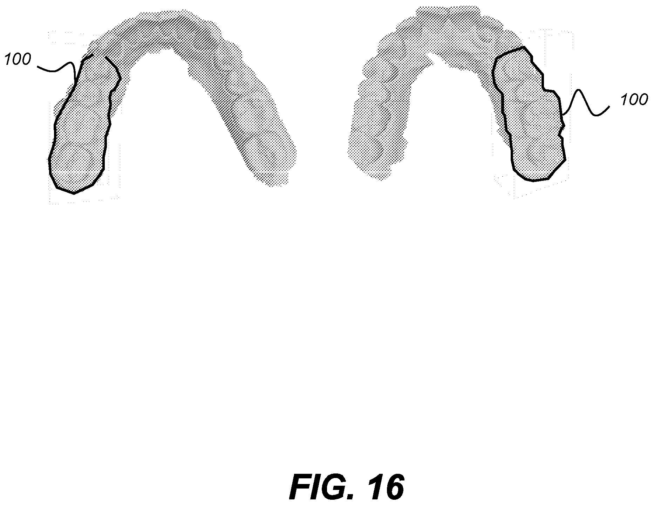

FIG. 16 shows registration of the bite impression data from facing occlusal surfaces with scanned data from the upper and lower arch. The relative position of impression material 100 used for the facing upper and lower bite imprints is shown.

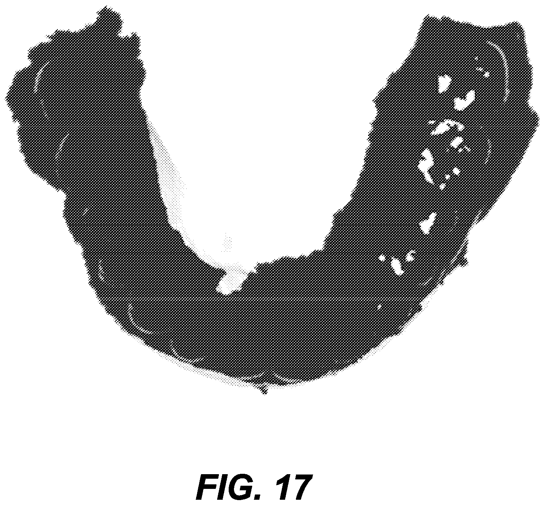

FIG. 17 shows the output of bite registration identified for an example not forming part of the invention, using the bite impression data.

Embodiments of the present disclosure include apparatus such as those shown in FIGs. 1 and 2 configured to acquire, process, and display image data from intraoral scanning and from partial impressions.

Consistent with an embodiment of the present invention, a computer program utilizes stored instructions that perform on image data that is accessed from an electronic memory. As can be appreciated by those skilled in the image processing arts, a computer program for operating the imaging system in an embodiment of the present disclosure can be utilized by a suitable, general-purpose computer system operating as a CPU as described herein, such as a personal computer or workstation. However, many other types of computer systems can be used to execute the computer program of the present invention, including an arrangement of networked processors, for example. The computer program for performing the method of the present invention may be stored in a computer readable storage medium. This medium may comprise, for example; magnetic storage media such as a magnetic disk such as a hard drive or removable device or magnetic tape; optical storage media such as an optical disc, optical tape, or machine readable optical encoding; solid state electronic storage devices such as random access memory (RAM), or read only memory (ROM); or any other physical device or medium employed to store a computer program. The computer program for performing the method of the present disclosure may also be stored on computer readable storage medium that is connected to the image processor by way of the internet or other network or communication medium. Those skilled in the art will further readily recognize that the equivalent of such a computer program product may also be constructed in hardware.

It should be noted that the term "memory", equivalent to "computer-accessible memory" in the context of the present disclosure, can refer to any type of temporary or more enduring data storage workspace used for storing and operating upon image data and accessible to a computer system, including a database, for example. The memory could be non-volatile, using, for example, a long-term storage medium such as magnetic or optical storage. Alternately, the memory could be of a more volatile nature, using an electronic circuit, such as random-access memory (RAM) that is used as a temporary buffer or workspace by a microprocessor or other control logic processor device. Display data, for example, is typically stored in a temporary storage buffer that is directly associated with a display device and is periodically refreshed as needed in order to provide displayed data. This temporary storage buffer is also considered to be a type of memory, as the term is used in the present disclosure. Memory is also used as the data workspace for executing and storing intermediate and final results of calculations and other processing. Computer-accessible memory can be volatile, non-volatile, or a hybrid combination of volatile and non-volatile types.

It will be understood that the computer program product of the present disclosure may make use of various image manipulation algorithms and processes that are well known. It will be further understood that the computer program product embodiment of the present disclosure may embody algorithms and processes not specifically shown or described herein that are useful for implementation. Such algorithms and processes may include conventional utilities that are within the ordinary skill of the image processing arts. Additional aspects of such algorithms and systems, and hardware and/or software for producing and otherwise processing the images or co-operating with the computer program product of the present disclosure, are not specifically shown or described herein and may be selected from such algorithms, systems, hardware, components and elements known in the art.

The invention has been described in detail and may have been described with particular reference to a suitable or presently preferred embodiment, but it will be understood that variations and modifications can be effected within the scope of the invention. The presently disclosed embodiments are therefore considered in all respects to be illustrative and not restrictive. The scope of the invention is indicated by the appended claims, and all changes that come within the meaning and range of the claims are intended to be embraced therein.

### 200010 Parts List

- 10.: Illumination array
- 12.: Patient
- 16.: Camera
- 20.: Surface
- 22.: Tooth
- 24.: Camera
- 28.: Scanner
- 30.: Sensor array
- 34.: Lens
- 40.: Sensing apparatus
- 42.: Sensor
- 46.: Light pattern
- 70.: Imaging apparatus
- 72.: Memory
- 74.: Display
- 75.: Computer
- 80.: Control logic processor
- 84.: Display
- 90.: Imaging apparatus
- 92.: 3D view
- 94.: Surface
- 96.: Margin
- 100.: Impression material

- 150.: Surface image

- S510.: Scanning step
- S520.: Surface image generation step
- S530.: ROI identification step
- S540.: Impression scanning step
- S550.: Registration step
- S560.: Merge step
- S570.: Display step

- S1210.: Scanning step
- S1220.: Surface image generation step
- S1230.: Impression scanning step
- S1240.: Surface image generation step
- S1250.: Registration step
- S1260.: Bite registration determination step
- S1270.: Display step

## Claims

1. A method for imaging a region of interest of patient (12) dentition (22), the method executed at least in part by a computer system (75) and comprising:
a) acquiring an intraoral scan directly from the patient dentition (22) and forming a 3D digital model of a first surface contour of the patient dentition (22) that includes the region of interest;
b) acquiring a physical impression (100) that includes at least the region of interest;
c) scanning the physical impression (100) and generating a second surface contour of the identified region of interest;
d) modifying the 3D digital model by combining a portion of the second surface contour with the first surface contour over the region of interest;
and
e) displaying the modified 3D digital model.

2. The method of claim 1, further comprising accepting one or more operator instructions that identify the region of interest.

3. The method of claim 2, wherein the one or more operator instructions are entered on a touch screen.

4. The method of claim 1, further comprising automatically detecting the region of interest.

5. The method of claim 4, wherein the region of interest is defined according to one or more boundaries of the physical impression (100).

6. The method of claim 1, wherein scanning the physical impression (100) comprises using an intraoral scanner or a desktop scanner (28).

7. The method of claim 1, wherein forming the 3D digital model comprises forming a point cloud or a mesh (150).

8. The method of claim 1, wherein combining comprises substituting one or more points of the 3D digital model with corresponding points of the second surface contour.

9. A method for imaging a region of interest of a dental arch, the method executed at least in part by a computer system (75) and comprising:
a) acquiring (S510) an intraoral scan directly from patient dentition and forming (S520) a 3D digital model of a first surface contour of the dental arch that includes the region of interest;
b) acquiring (S540) a partial physical impression (100) of a portion of the dental arch that defines (S530) the region of interest;
c) scanning the acquired partial physical impression (100) and generating (S550), from the scanned partial physical impression (100), a second surface contour of the defined region of interest;
d) modifying (S560) the 3D digital model by replacing a portion of the first surface contour with the second surface contour over the defined region of interest;
and
e) displaying (S570) the modified 3D digital model.

10. An imaging apparatus (90) for imaging a dental arch of a patient (12), comprising:
a scanner (28) that is energizable to acquire a sequence of reflectance images of the patient dental arch directly from patient dentition (22) and of a partial physical impression (100) obtained from a portion of the dental arch;
a logic processor (80) that is programmed with instructions:
(i) to acquire the sequence of reflectance images of the patient dental arch;
(ii) to form a 3D digital model of a first surface contour of the patient dental arch that includes the region of interest;
(iii) to acquire the sequence of images of the partial physical impression (100);
(iv) to form a 3D digital model of the partial physical impression (100) as a second surface contour of the region of interest ;
(v) to combine the 3D digital models of the first and second surface contour to form a hybrid 3D digital model for display;
and
a display (74) that is in signal communication with the logic processor (80) and
configured to display a rendering of the hybrid 3D model.

## Patentansprüche

1. Verfahren zum Abbilden eines relevanten Bereichs des Gebisses (22) eines Patienten (12), wobei das Verfahren zumindest teilweise von einem Computersystem (75) ausgeführt wird und Folgendes umfasst:
a) Erfassen eines Intraoralscans direkt vom Gebiss (22) des Patienten und Bilden eines 3D-Digitalmodells einer ersten Oberflächenkontur des Gebisses (22) des Patienten, die den relevanten Bereich einschließt;
b) Gewinnen eines physischen Abdrucks (100), der zumindest den relevanten Bereich einschließt;
c) Scannen des physischen Abdrucks (100) und Generieren einer zweiten Oberflächenkontur des identifizierten relevanten Bereichs;
d) Modifizieren des 3D-Digitalmodells durch Kombinieren eines Abschnitts der zweiten Oberflächenkontur mit der ersten Oberflächenkontur über den relevanten Bereich;
und
e) Anzeigen des modifizierten 3D-Digitalmodells.

2. Verfahren nach Anspruch 1, wobei das Verfahren weiter das Annehmen einer oder mehrerer Bedieneranweisungen, die den relevanten Bereich identifizieren, umfasst.

3. Verfahren nach Anspruch 2, wobei die eine oder mehrere Bedieneranweisungen auf einem Touchscreen eingegeben werden.

4. Verfahren nach Anspruch 1, wobei das Verfahren weiter das automatische Detektieren des relevanten Bereichs umfasst.

5. Verfahren nach Anspruch 4, wobei der relevante Bereich entsprechend einer oder mehrerer Grenzen des physischen Abdrucks (100) definiert ist.

6. Verfahren nach Anspruch 1, wobei das Scannen des physischen Abdrucks (100) das Verwenden eines Intraoralscanners oder eines Desktop-Scanners (28) umfasst.

7. Verfahren nach Anspruch 1, wobei das Bilden des 3D-Digitalmodells das Bilden einer Punktwolke oder eines Netzes (150) umfasst.

8. Verfahren nach Anspruch 1, wobei das Kombinieren das Ersetzen eines oder mehrerer Punkte des 3D-Digitalmodells durch entsprechende Punkte der zweiten Oberflächenkontur umfasst.

9. Verfahren zum Abbilden eines relevanten Bereichs eines Zahnbogens, wobei das Verfahren zumindest teilweise von einem Computersystem (75) ausgeführt wird und Folgendes umfassend:
a) Erfassen (S510) eines Intraoralscans direkt vom Gebiss des Patienten und Bilden (S520) eines 3D-Digitalmodells einer ersten Oberflächenkontur des Zahnbogens, die den relevanten Bereich einschließt;
b) Gewinnen (S540) eines teilweisen physischen Abdrucks (100) eines Abschnitts des Zahnbogens, der (S530) den relevanten Bereich definiert;
c) Scannen des erlangten teilweisen physischen Abdrucks (100) und Generieren (S550), aus dem gescannten teilweisen physischen Abdruck (100), einer zweiten Oberflächenkontur des definierten relevanten Bereichs;
d) Modifizieren (S560) des 3D-Digitalmodells durch Ersetzen eines Abschnitts der ersten Oberflächenkontur durch die zweite Oberflächenkontur über den definierten relevanten Bereich;
und
e) Anzeigen (S570) des modifizierten 3D-Digitalmodells.

10. Abbildungseinrichtung (90) zum Abbilden eines Zahnbogens eines Patienten (12), umfassend:
einen Scanner (28), der aktivierbar ist, um eine Sequenz von Reflexionsbildern des Zahnbogens des Patienten direkt vom Gebiss (22) des Patienten und von einem teilweisen physischen Abdruck (100), der aus einem Abschnitt des Zahnbogens erhalten wird, zu erfassen;
einen Logikprozessor (80), der mit Anweisungen programmiert ist, um:
(i) die Sequenz von Reflexionsbildern des Zahnbogens des Patienten zu erfassen;
(ii) ein 3D-Digitalmodell einer ersten Oberflächenkontur des Zahnbogens des Patienten zu bilden, die den relevanten Bereich einschließt;
(iii) die Sequenz von Bildern des teilweisen physischen Abdrucks (100) zu gewinnen;
(iv) ein 3D-Digitalmodell des teilweisen physischen Abdrucks (100) als eine zweite Oberflächenkontur des relevanten Bereichs zu bilden;
(v) die 3D-Digitalmodelle der ersten und der zweiten Oberflächenkontur zu kombinieren, um ein hybrides 3D-Digitalmodell zur Anzeige zu bilden; und
eine Anzeige (74), die mit dem Logikprozessor (80) in Signalverbindung steht und ausgebildet ist, eine Darstellung des hybriden 3D-Digitalmodells anzuzeigen.

## Revendications

1. Procédé d'imagerie d'une région d'intérêt de la dentition (22) d'un patient (12), le procédé étant mis en œuvre au moins en partie par un système informatique (75) et comprenant :
a) l'acquisition d'un balayage intra-buccal directement à partir de la dentition (22) du patient, et le formage d'un modèle numérique tridimensionnel d'un premier contour de surface de la dentition (22) du patient, qui inclut la région d'intérêt ;
b) l'acquisition d'une empreinte physique (100) qui inclut au moins la région d'intérêt ;
c) le balayage de l'empreinte physique (100) et la génération d'un deuxième contour de surface de la région d'intérêt identifiée ;
d) la modification du modèle numérique tridimensionnel em combinant une portion du deuxième contour de surface avec le premier contour de surface sur la région d'intérêt ; et
e) l'affichage du modèle numérique tridimensionnel modifié.

2. Procédé selon la revendication 1, comprenant en outre l'acceptation d'une ou plusieurs instructions d'opérateur qui identifient la région d'intérêt.

3. Procédé selon la revendication 2, dans lequel les une ou plusieurs instructions d'opérateur sont saisies sur un écran tactile.

4. Procédé selon la revendication 1, comprenant en outre la détection automatique de la région d'intérêt.

5. Procédé selon la revendication 4, dans lequel la région d'intérêt est définie selon une ou plusieurs limites de l'empreinte physique (100).

6. Procédé selon la revendication 1, dans lequel le balayage de l'empreinte physique (100) comprend l'utilisation d'un scanner intra-buccal ou d'un scanner de bureau (28).

7. Procédé selon la revendication 1, dans lequel le formage du modèle numérique tridimensionnel comprend le formage d'un nuage de points ou d'un maillage (150).

8. Procédé selon la revendication 1, dans lequel la combinaison comprend la substitution d'un ou plusieurs points du modèle numérique tridimensionnel par des points correspondants du deuxième contour de surface.

9. Procédé d'imagerie d'une région d'intérêt d'une arcade dentaire, le procédé étant mis en œuvre au moins en partie par un système informatique (75) et comprenant :
a) l'acquisition (S510) d'un balayage intra-buccal directement à partir de la dentition d'un patient et le formage (S520) d'un modèle numérique tridimensionnel d'un premier contour de surface de l'arcade dentaire qui inclut la région d'intérêt ;
b) l'acquisition (S540) d'une empreinte physique partielle (100) d'une portion de l'arcade dentaire qui définit (S530) la région d'intérêt ;
c) le balayage de l'empreinte physique partielle (100) acquise et la génération (S550), à partir de l'empreinte physique partielle (100) balayée, d'un deuxième contour de surface de la région d'intérêt définie ;
d) la modification (S560) du modèle numérique tridimensionnel par remplacement d'une portion du premier contour de surface par le deuxième contour de surface sur la région d'intérêt définie ;
et
e) l'affichage (S570) du modèle numérique tridimensionnel modifié.

10. Appareil d'imagerie (90) destiné à imager une arcade dentaire d'un patient (12), comprenant :
un scanner (28) qui peut être alimenté pour acquérir une séquence d'images de réflexion de l'arcade dentaire du patient directement à partir de la dentition (22) du patient et d'une empreinte physique partielle (100) obtenue à partir d'une portion de l'arcade dentaire ;
un processeur logique (80) qui est programmé avec des instructions :
(i) pour acquérir la séquence d'images de réflexion de l'arcade dentaire du patient ;
(ii) pour former un modèle numérique tridimensionnel d'un premier contour de surface de l'arcade dentaire du patient qui inclut la région d'intérêt ;
(iii) pour acquérir la séquence d'images de l'empreinte physique partielle (100) ;
(iv) pour former un modèle numérique tridimensionnel de l'empreinte physique partielle (100) en tant que deuxième contour de surface de la région d'intérêt ;
(v) pour combiner les modèles numériques tridimensionnels des premier et deuxième contours de surface afin de former un modèle numérique tridimensionnel hybride en vue de l'affichage ;
et
un écran (74) qui est en communication de signal avec le processeur logique (80) et qui est configuré pour afficher un rendu du modèle tridimensionnel hybride.
